Europäisches Patentamt

(19) European Patent Office

Office européen des brevets

(11) Publication number: **0 052 995**

**B1**

(12) **EUROPEAN PATENT SPECIFICATION**

(45) Date of publication of patent specification: **13.03.85**

(21) Application number: **81305424.4**

(22) Date of filing: **17.11.81**

(51) Int. Cl.⁴: **H 05 G 1/60,** A 61 B 6/02, G 03 B 42/02

(54) X-ray apparatus.

(30) Priority: **20.11.80 JP 163934/80**

(43) Date of publication of application:
**02.06.82 Bulletin 82/22**

(45) Publication of the grant of the patent:
**13.03.85 Bulletin 85/11**

(84) Designated Contracting States:
**DE FR GB NL**

(56) References cited:
**DE-A-1 929 894**
**DE-A-2 062 430**
**DE-B-1 262 772**
**GB-A-2 034 967**
**US-A-3 244 878**
**US-A-3 424 901**
**US-A-3 684 354**

(73) Proprietor: **Kabushiki Kaisha Toshiba
72, Horikawa-cho Saiwai-ku
Kawasaki-shi Kanagawa-ken 210 (JP)**

(72) Inventor: **Kurihara, Tetsurou Patent Division
Tokyo Shibaura Denki K.K. 72 Horikawa-cho
Saiwai-ku Kawasaki-shi Kanagawa-ken (JP)**
Inventor: **Nishio, Kohsaku Patent Division
Tokyo Shibaura Denki K.K. 72 Horikawa-cho
Saiwai-ku Kawasaki-shi Kanagawa-ken (JP)**

(74) Representative: **Batchellor, John Robert et al
BATCHELLOR, KIRK & EYLES 2 Pear Tree Court
Farringdon Road
London EC1R 0DS (GB)**

Courier Press, Leamington Spa, England.

## Description

This invention relates to X-ray apparatus and is aimed at providing such apparatus capable of performing steroscopic, or mono-scopic X-ray cinematography, or X-ray fluoroscopy.

In the field of X-ray examination, and particularly in that utilising angiography, there is a great demand to enable an object, such as a patient, to be viewed three-dimensionally. Previously, single directional radiography has generally been performed in the diagnosis and treatment of, for example, congenital heart disease, valvular heart disease, and hepatic artery disease of the heart, where cardiovascular angiography is feasible; uni-directional radiography however shows nothing of a three-dimensional object other than a single plane, and it is extremely difficult to view a unidirectional image stereoscopically.

If two-directional radiography is performed in an attempt to improve the examination of the three-dimensional object, only two plane images which differ only in radiographic direction are obtained and, again, a stereoscope is unobtainable.

In cardiovascular angiography, X-ray fluoroscopy is performed, while a contrasting medium is injected in the object to be observed and the resulting fluoroscopic images are photographed with a cinecamera. However since, in the conventional system, a different X-ray tube and a different cinecamera is used for each radiographic direction, the resulting images in each direction is a plane image. Consequently, it is difficult to obtain precise observation of cardiovascular systems and the like because of the impossibility of stereoscopic viewing. From DE—B—1 262 772 X-ray equipment capable of performing stereoscopic cinematography is known, the equipment comprising a pair of points from which X-rays are independently radiated to create an X-ray image of an object; means for converting the X-ray image to a corresponding optical image; an optical system arranged to receive and transmit the optical image, that system including a half-mirror which is substantially equally reflective and transmissive; a first cinecamera arranged to receive the image transmitted from the converting means through the half-mirror; a second cinecamera arranged to receive the image transmitted from the converting means and reflected by the half-mirror, the two cinecameras having shutters which are out of phase with one another; and a radiographic control device controlling the photographing operation of the cinecameras and that for stereoscopic cinematography X-rays are radiated from both focal points alternately in synchronism with the alternately open shutters for both cinecameras. From DE—A—2 062 430 an X-ray quipment for stereoscopic cinematography is known comprising means alternately emitting X-rays from two points, a converter, an optical system, a cinecamera and a television camera arranged to receive the image from the converter.

From GB—A—2 034 967 a stereoscopic X-ray equipment is known comprising an X-ray tube having at least a pair of focal points, from which X-rays are independently radiated. From US—A—3 424 901 X-ray equipment capable of performing stereoscopic cinematography is known, the optical system of which comprises a small light deflector to receive light from the converting means.

In the present invention, an X-ray tube designed to radiate X-rays independently from at least two focal points is employed with an optical system for independently projecting images derived from those focal points, so that stereographs may be obtained.

Thus, the present invention provides X-ray apparatus capable of performing stereoscopic cinematography, single cinematography and fluoroscopy, the equipment comprising an X-ray tube having at least a pair of focal points from which X-rays are independently radiated to create an X-ray image of an object; means for converting the X-ray image to a corresponding optical image; an optical system arranged to receive and transmit the optical image, that system including a half-mirror which is substantially equally reflective and transmissive and which is movable between an operative position in which it intercepts the light path of the optical image and an inoperative position, a fully reflective mirror which is movable between an operative position in which it intercepts the light path and an inoperative position; and a small light deflector to receive light from the converting means; a first cinecamera arranged to receive the image transmitted from the converting means through the half-mirror; a second cinecamera arranged to receive the image transmitted from the converting means and reflected by the half-mirror in its operative position, the two cinecameras having shutters which are out of phase with one another; a television camera means arranged to receive the image from the converting means either via the fully reflective mirror in its operative position or via the small deflector; a mode setting device for selecting one of the stereoscopic cinematography, single cinematography and fluoroscopy modes; and a radiographic control device for controlling the positioning of the half mirror and the fully reflective mirror according to the selected mode so that the half-mirror is brought into its operative position for stereographic cinematography to direct the image to both cinecameras, the half-mirror is brought into its inoperative position for single cinematography, and the fully reflective mirror is brought into operative position for fluoroscopy to direct the image to the television camera means, the radiographic control device further controlling the photographing operation of the cinecameras according to the selected mode, and controlling the X-ray tube so that, for stereoscopic cinematography, X-rays are radiated from both focal points alternately in synchronism with the alternately open shutters of the two cinecameras, and, for single cinematography, X-rays are radiated intermittently from one focal point in synchronism with the open state of the shutter of one cinecamera.

By a "half-mirror" is meant a mirror which is part-transmissive and part-reflective.

The invention will be more readily understood by way of example from the following description of X-ray apparatus in accordance therewith, reference being made to the accompanying drawings, in which

Figure 1 is a block diagram, schematically illustrating the apparatus;

Figure 2(a) is a sectional view of the optical system of Figure 1;

Figure 2(b) is a section on the line A—A' of Figure 2(a);

Figure 3 is a circuit diagram illustrating one form of the X-ray tube switching controller of Figure 1;

Figure 4(a) to (f) are timing diagrams explaining the operational sequence of the apparatus;

Figure 5 is a sectional view of a modified form of the optical system of Figures 2(a) and (b);

Figure 6(a) is a sectional view of another modification of the topical system of Figures 2(a) and (b); and

Figure 6(b) is a section on the line A—A' of Figure 6(a).

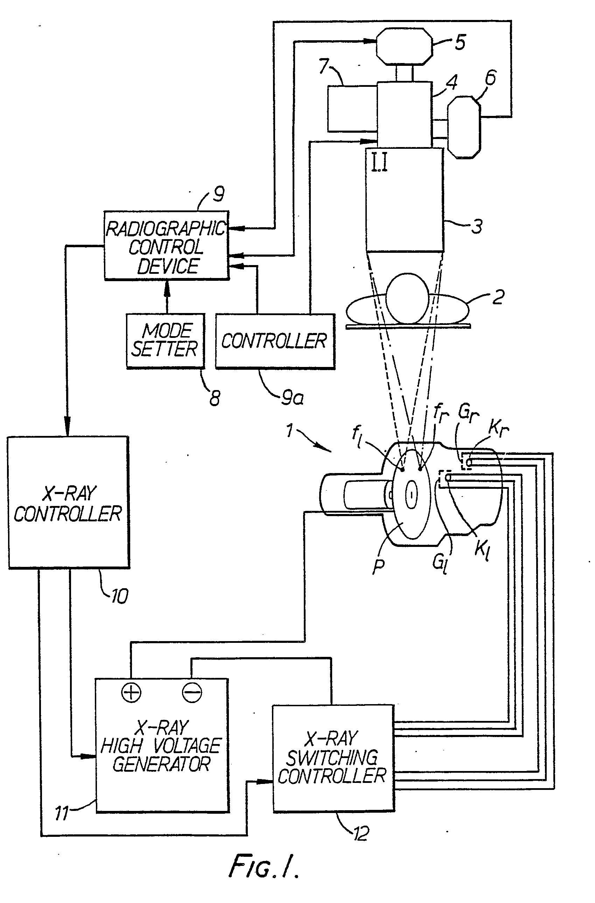

Referring to Figure 1, an X-ray tube 1 for stereoscopic radiography has a conical trapezoid shaped rotating anode P, and two cathodes Kr K/ spaced apart by a distance corresponding to the average human inter-ocular distance, and facing the oblique part of anode P. Hereinafter, the suffixes r and / used in conjunction with the reference numerals of the drawings relate to the right and left eyes, respectively. Grids Gr and G/ are interposed between the cathodes Kr and K/ and the rotating anode P and are employed for X-ray exposure control. The potentials on the two grids are alternately altered to cause X-ray radiation alternatively from the corresponding anode parts during the times when electrons are emitted from the respective cathodes K/ and Kr. The two beams of electrons from the cathodes impinge upon the portions of the oblique anode P facing the cathodes, to define two focal points f1 and fr from which a pair of X-ray beams are radiated. In this way, two X-ray beams are generated, spaced laterally at the inter-ocular distance. The X-ray tube 1 as described above is disclosed in U.S. Patent No. 4287420, for example.

An object 2 such as a patient is interposed between the X-ray tube 1 and an image intensifier 3 which receives on its input face the X-ray beams after passing through the object 2, and forms light images. Optical system 4 is connected to the output 3a of intensifier 3 and projects the optical output image as will be described. Cinecameras 5 and 6 are attached respectively to the end and to one side of the optical system 4 to photograph optical images directed thereto to the optical system. The cinecameras 5 and 6 are synchronously driven, so that their shutters open alternately, and so that the shutters are never open together. Generally each shutter is formed by a disc having a window at a given angular position; by rotating the shutter one image per frame is photographed on the cinefilm, the movement of which is synchronised with the shutter movement. In that case, when stereoscopic radiography is to be performed, the shutters of the cinecameras are arranged 180° out of phase. Cinecameras 5 and 6 issue a series of shutter motion detection signals when their shutters are open, the outputs being employed for exposure control of the X-ray tube 1. A television camera 7 converts the optical image from the intensifier 3 to an electrical image signal and it delivers the latter signal to a television monitor (not shown).

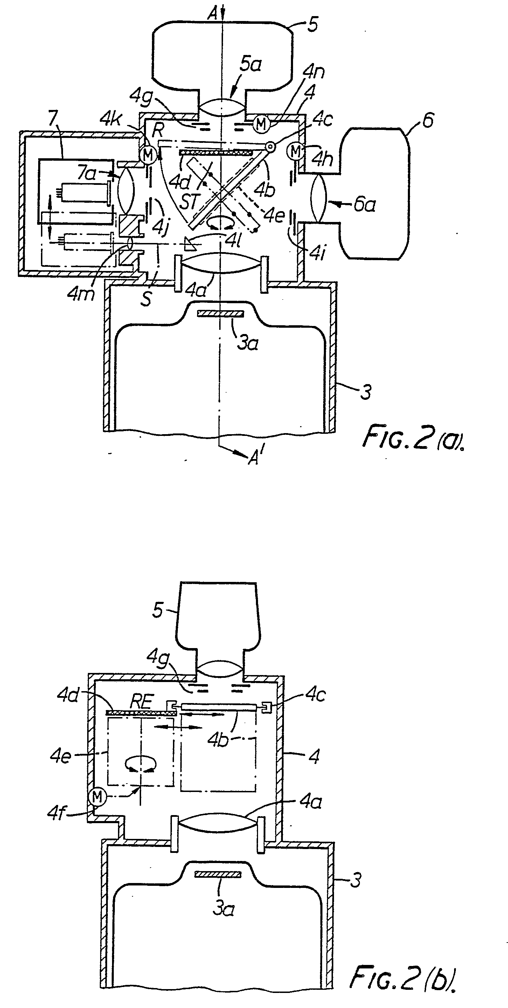

In Figures 2(a) and 2(b) the optical system 4 and the optical system peripherals are shown in detail. In the figure, the optical system 4 comprises a light-impervious enclosure attached to the image intensifier 3 and having a lens 4A adjacent the outward face of intensifier 3. Cinecamera 5 with its lens 5a is attached to the other end of the enclosure, coaxially with lens 4a. Cinecamera 6 and television camera 7 are attached to the optical system 4 so as to face one another with their optical axes intersecting the optical axis of the lens 4a at an angle of 90°.

A half-mirror 4b is pivotally mounted within the enclosure and, in the position shown in Figure 2(a) is transverse to the axes of both lenses 4a, 6a in order to divert the image from the intensifier 3 and the lens 4a. This half-mirror 4b has a reflection rate of 50% and a transmission rate of 50%. The half-mirror 4b is hinged at its end by a supporting pin 4c enabling it to move from the first position shown in Figure 2(a) to the position shown in Figure 2(b) where it lies parallel to lens 5a, under the operation of drive means (not shown). These positions will be referred to hereinafter as "the set position" 5 and "the shunted position" R, respectively.

A fully reflective mirror 4d can be translated from a side shunted position shown in Figure 2(b) to an operative position in front of half-mirror 4b when the latter is in shunted position R, as shown in Figure 2(a). In the latter position, fully reflective mirror 4d is normal to the optical axis of the lens 4a. Mirror 4d is usually in the shunted position (position RE in Figure 2(b)) out of the optical path of the lens 4a and executes a parallel movement to the operative position (position ST in Figure 2(a)) in the optical path when it is required to be used. Such operation is performed by drive manipulating means (not shown).

Provided in the incident side of the fully reflective mirror 4d is a mirror unit which includes second half-mirror 4e having an inclination of 45° to the optical axis of the lens 4a, a reflection rate of 90% and a transmission rate of 10%. This mirror unit is usually in the shunted position indicated in Figure 2(b), out of the optical axis of the lens 4a in similar manner to the fully reflective mirror 4d and is brought into the optical path of the lens 4a for fluoroscopy or single mode cinematography. The mirror unit is independent of the fully reflective mirror 4d and is moved between the shunted position and the operative position by drive manipulating means (not shown). Also, half-mirror 4e can be rotated about its centre line by a motor 4f housed in the mirror unit so that the reflected light from the half-mirror 4e can be directed either to the cinecamera 6 or to television camera 7.

An optical diaphragm 4i in front of lens 6a is controlled by a motor 4h to vary the amount of incident light entering lens 6a of the cinecamera 6. This optical diaphragm 4i functions so that, when the half-mirror 4b is shunted and half-mirror 4e is brought into operative position, the diaphragm closes to maintain the light to the cinecamera 6 at a uniform optimum value. Conversely, when half-mirror 4e is moved into the shunted position and half-mirror 4b is brought into the set position, optical diaphragm 4i opens, again to maintain constant the level of light directed to cinecamera 6. Diaphragm 4i is also used as a shutter for completely interrupting the incident light when cinecamera 6 is not in use. In this case, the optical diaphragm of the cinecamera 6 itself may be used instead of the diaphragm 4i and if an external control or automatic control of the camera diaphragm is available.

A diaphragm 4g is provided for the cinecamera 5, while a diaphragm 4j adjusts the incident light level to the television camera 7 in response to the movement of the half-mirrors 4b and 4e in and out of the light path. The diaphragm 4j is actuated by a motor 4k.

Since half-mirror 4b has the rotating function described, only minimum space is needed for its movement from the set position S to the shunted position R. For the same reason, the half-mirror 4e or the fully reflective mirror 4d can be inserted in the optical system, while mirror 4b is shunted, without further movement of the latter. Because the space needed for the mirrors is kept to a minimum, the two cinecameras 5 and 6 can readily be located at equal distances from the set position S of half-mirror 4b. As a result, it is possible to equalize the individual path lengths from the lens 4a to the cinecameras 5 and 6, and to keep those distances short. Because of the short path lengths, the light attenuation which is usually associated with a primary lens is kept within reasonable bounds. Also as the cinecameras 5 and 6 are equidistant from the primary lens 4a, their optical conditions are identical and each receives exactly the same image for photographing.

A small prism 4l for light distribution is disposed near one edge of lens 4a at the emitting side thereof. Prism 4l diverts a part of the light from lens 4a parallel to, but to one side of, television camera 7, when in the full line position of Figure 2(a). Television camera can be moved sideways by an appropriate mechanism to the position shown in dotted line in Figure 2(a) to bring its optical axis into alignment with prism 4l.

A small diameter lens 4m is located on the output optical axis of the prism 4l and in front of the television camera 7, when in the dotted line position of Figure 2(a). As will be appreciated, the television camera 7 includes no image forming part and uses lens 4m or lens 7a according to the position the camera occupies.

Position detectors (not shown) detect the positions of mirrors 4b, 4d and 4e and transmits signals representing those positions.

Reverting to Figure 1, a mode setter 8 determines the mode in which the apparatus is to operate, i.e. whether in the stereoscopic cinematography mode, the single cinematography mode or the fluoroscopy mode. A radiographic control device 9 which receives signals from mode setter 8, the cinecamera shutter drives and the mirror detectors delivers signals to a controller 9a which in turn controls the motors 4f, 4h, 4k and 4n, the drives for selectively moving the mirrors, the drives for cinecameras 5 and 6 when stereoscopy mode is selected, the drive of cinecamera 6 only when the mono-scopic mode is selected, and the X-ray tube 1.

An X-ray controller 10 controlled by control device 9 emits signals to X-ray high voltage generator 11 to set the X-ray tube voltage and current according to whether the fluoroscopy mode or the radiographic mode is selected. In addition, controller 10 emits signals to X-ray switching controller 12 which in turn controls the emission of the beams from the focal points fr and fl. Thus, the "right" beam from focal point fr is generated when the shutter of cinecamera 5 is open and the "left" X-ray beam from focal pint fl is generated when the shutter of cinecamera 6 is open.

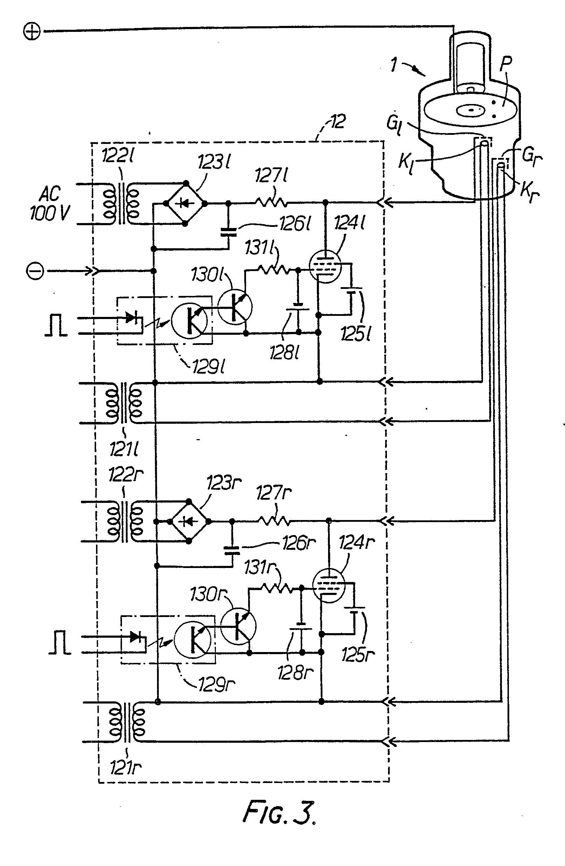

The X-ray tube switching controller 12 is shown in greater detail in Figure 3, in which the signs + and − designate input terminals which receive respectively the positive and negative high voltage outputs from the generator 11. Controller 12 includes two identical control circuits, one for the "right" X-ray beam and the other for the "left" X-ray beam. As the two circuits are identical, only one will be described, although both are shown in Figure 3 with the suffixes r and l.

A filament heating transformer 121 receives an output from generator 11 and generates a heating voltage corresponding to its input, that voltage being applied to the corresponding cathode K of the X-ray tube 1. Transformer 122 for producing a grid potential is connected to a power source and generates a variable voltage at its output. Rectifier circuit 123 performs full rectification to obtain a d.c. voltage of negative polarity. Tetrode 124 for the exposure switching control of the X-ray tube is connected between the corresponding grid and cathode of the X-ray tube 1. A power supply source 125 is connected between the cathode and second grid of tetrode 124 and applies to the second grid a positive bias to bring the internal resistance of the tetrode to an optimum value. Condenser 126 is connected between the output terminals of the rectifier circuit 123 and a resistor 127 is connected between the rectifying circuit 123 and the corresponding grid of tube 1. Potential source 128 is connected between the cathode and first grid of tetrode 124 to bias negatively the first grid to an optimum value.

A photo-coupler 129 is rendered active on receipt of the X-ray exposure switching control output from controller 10 and its output activates a switching transistor 130 having its emitter connected to the negative terminal or potential source 128 through resistor 131 and its collector to the positive terminal of that source.

The high voltage output from generator 11 is applied between the anode and the cathodes of tube 1, being applied to the cathodes through the filament heating transformers 121. The negative potential of rectifying circuit 123 is applied to the respective grid of tube 1 through resistor 127, and the positive potential to the respective cathode, so as to apply negative bias to the grid G and to hold the tube 1 in the cut-off state. Although the output voltage of rectifying circuit 123 is supplied to tetrode 124 as the tube voltage, the tetrode is inversely biased between its primary grid and cathode and is normally in its cut-off state.

Subsequently, when photo-coupler 129 receives a control output from controller 10, transistor 130 is rendered conducting, to close the loop formed by bias source 28, transistor 130, and resistor 131. The resulting voltage drop across resistor 131 increases the potential on the first grid of tetrode 124 and the tetrode is rendered conducting. Current now flows through resistor 127, the potential on the respective grid G of tube 1 is increased, electrons flow from the respective cathode K to the anode P, and an electron beam is emitted from the respective focal point f/ or fr.

When the input to the photo-coupler 129 disappears, transistor 130 is rendered non-conducting causing tetrode 124 to be cut-off. Negative bias is again applied to the respective grid K of tube 1 and the X-ray beam from the correponding focal point of the anode ceases. In this way, it is possible to control the X-ray exposures corresponding to the right and left eyes by controlling the photo-couplers 129r and 129/ by the X-ray controllers 10.

The operation of the optical system will now be described:

Mode setter 8 is initially operated to select one of the three modes.

Stereoscopic Cinematography

Mode setter 8 applies an appropriate instruction to control device 9 which in turn issues an appropriate instruction to controller 9a. Controller 9a sends control signals to the optical system 4 to cause fully reflective mirror 4d and half-mirror 4e to be moved into their shunted positions, the half-mirror 4b into its set position S and the television camera 7 into the position facing prism 4/. As half-mirror 4b is now in the optical path of the primary lens 4a, light transmitted through the primary lens 4a is directed to cinecameras 5 and 6, while a small part of the light passes to the television camera 7.

The radiographic control device 9 delivers a driving output to each of the motors 4h, 4k and 4n to adjust each of the optical diaphragms 4i, 4j and 4g in accordance with the incident light levels. As the light levels and the degree of cut-off of the diaphragms are known in advance, the diaphragms may be automatically adjusted to preset values according to the selected mode. The optical diaphragms 4g, 4i and 4j are used as optical shutters when the respective cameras are not in use. In particular, the optical diaphragm 4g of cinecamera 5 may have a fixed opening or be manually set because the cinecamera 5 is used only in the stereoscopic cinematography.

The optical system 4 has now been set for performing stereoscopic cinematography.

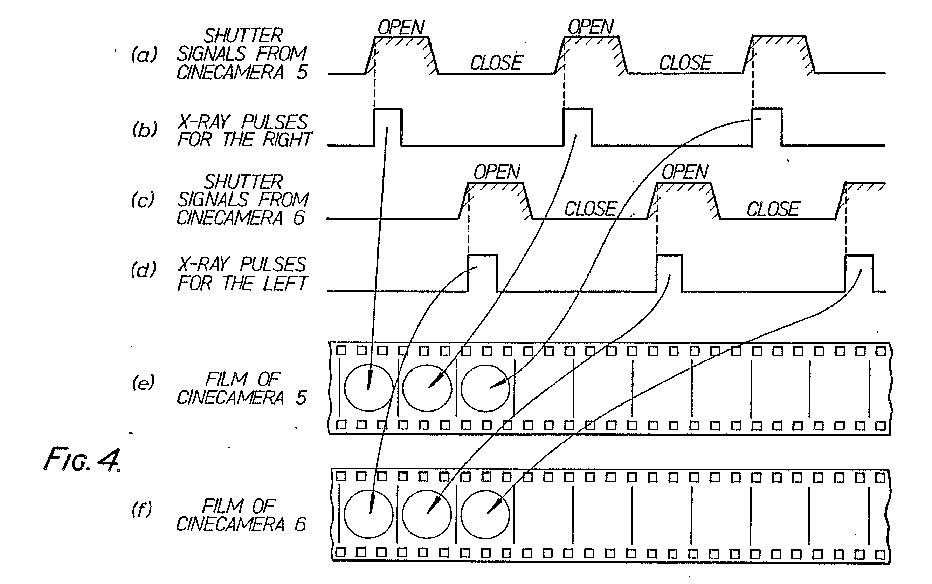

Subsequently, the radiographic control device 9 controls the X-ray controller 10 to set the X-ray tube voltage and current for radiography. If a radioagraphy start command is now applied to the radiographic control device 9, it delivers drive signals to the cinecameras 5 and 6, respectively. The shutters of cinecameras 5 and 6 are now driven; their rotations are synchronised but are 180° out of phase so that the shutters are never open at the same time as shown in Figures 4(a) and (c). A signal synchronised with the shutter of camera 5 is issued when the shutter opens and is applied to the radiographic control device 9 which then generates an X-ray exposure switching control output.

Now, if cinecamera 5 is used for the right side in stereoscopy and cinecamera 6 for the left, the radiographic control device 9 applies an X-ray exposure switching control output to the X-ray controller 10 to effect a "right" X-ray exposure after receiving the detecting signal from the cinecamera 5. The X-ray controller 10, thereby, applies that X-ray exposure switching control output to the photo-coupler 129r in the control circuit of Figure 3 of X-ray tube switching controller 12.

As a result, the "right" tetrode 124r conducts as previously described, electrons are emitted toward anode P from the "right" cathode Kr of the X-ray tube 1 to which the output from the X-ray high voltage generator 11 is supplied and X-rays are emitted as shown in Figure 4(b) from the point fr during the time for which the photo-coupler 129r is activated.

The resultant X-ray image of the object 2 is formed on the input face of the image intensifier 3 and converted by it to an optical image which is supplied to the optical system device 4. The optical image incident to the optical system 4 is received by half-mirror 4b which directs about 50% of the light to cinecamera 5 so that the "right" image of the object 2 is photographed in the cinecamera 5 the shutter of which is opened as shown in Figure 4(e). Meanwhile although the unreflected light of about 50% passes through mirror 4b to cinecamera 6, the image is not photographed as the shutter of camera 6 is closed.

At the same time, a very small amount of the light through lens 4a is directed to the small television lens 4m by the prism 4l. Since the television camera 7 has been moved into alignment with lens 4m, the incident light is delivered to the television camera 7, an image being displayed on the television monitor. Although prism 4l and television lens 4m are small, and the light received by the television camera is correspondingly small, observable images are obtained, because the X-ray intensity in the radiographic condition is hundreds of times greater than in the fluoroscopic condition.

Subsequently, when the shutter of cinecamera 5 is closed and the shutter of cinecamera 6 is opened as shown in Figure 4(c), an appropriated detection signal is sent to the radiographic control device 9. As a result, the radiographic control device 9 issues an X-ray exposure switching control output for the left side to cause the X-ray controller 10 to apply a signal to photo-coupler 129l of X-ray tube switching controller 12. Tetrode 124l is turned on so that the inverse bias on the "left" grid Gl of the X-ray tube 1 is released and electrons are emitted toward the anode P from the left cathode Kl. Therefore, X-rays are emitted as shown in Figure 4(d) from the point fl for the period for which photo-coupler 129l is energised.

As before, an optical image of the subject 2 is delivered to the mirror 4b and distributed about equally to cinecameras 5 and 6. At the same time a small proportion of the light from lens 4a is directed to television camera 7 by prism 4l. The image is not photographed in cinecamera 5 which has its shutter in the closed condition, and is photographed only in cinecamera 6 as shown in Figure 4(f). The television camera 7 displays the image on the television monitor as before.

As described above, the "right" X-ray image is photographed in cinecamera 5 while the "left" X-ray image is photographed in cinecamera 6. The described operations are alternately repeated for the right and the left until a radiographic stop instruction is issued.

Single Cinematography

In the single cinematography mode, an instruction for single cinematography is delivered to the controller 9a from the radiographic control device 9 so that controller 9a causes half-mirror 4b to move into the shunt position R, the fully reflective mirror 4d to move into the set position ST and the half-mirror 4e to move into the optical pass of the primary lens 4a in front of the fully reflective mirror 4d. Furthermore, the controller 9a causes television camera 7 to be moved into alignment with television lens 7a.

Outputs from radiographic control device 9 are simultaneously applied to motors 4h and 4k to adjust the optical diaphragms 4i and 4j to preset diaphragm values corresponding to the light level from half-mirror 4e. As cinecamera 5 is unused for single cinematography its optical diaphragm 4g is closed.

After it has been transferred into the optical axis of lens 4a, half-mirror 4e is rotated by motor 4f so as to reflect the incident light to cinecamera 6, the necessary control being performed by controller 9a. On completion of the setting of mirror 4e, radiographic control device 9 supplies a drive output to cinecamera 6 and the grid Gl of X-ray tube 1 is switched on and off as previously described in synchronism with the shutter of cinecamera 6, in order to perform X-ray exposures.

The resultant "left" X-ray image is distributed by mirror 4e, 90% of the light being directed to cinecamera 6 and the light image of the object being photographed by cinecamera 6. Although the remaining 10% of the light goes straight through the half-mirror 4e, it is fully reflected by mirror 4d and re-directed toward the half-mirror 4e. 90% of the reflected light from mirror 4d is again reflected by half-mirror 4e to lens 7a and television camera 7 for the display of the image on the monitor as a fluoroscopic image. Light cannot reach cinecamera 5 because the fully reflective mirror 4d is in intercepting position in front of lens 5a.

Fluoroscopy

In a fluoroscopy mode, a control instruction for fluoroscopy is issued to controller 9a from the radiographic control device 9 and motor 4f is caused to be driven to rotate half-mirror 4e in the mirror unit into the position in which it directs reflected light along the optical axis of the television lens 7a. The radiographic control device 9 simultaneously applies a driving output to motor 4k to adjust optical diaphragm 4j of the television lens 7a for optimum exposure corresponding to its incident light level.

The radiographic control device 9 also controls X-ray controller 10 to set the X-ray tube current and voltage for fluoroscopy. Subsequently X-ray controller 10 sets the X-ray high voltage generator in the fluoroscopy condition. After that, following the delivery of an X-ray fluoroscopy instruction, a signal releasing the inverse bias on the "right" or "left" grid of the X-ray tube 1 is delivered to the X-ray tube switching controller 12 through the X-ray controller 10, to effect X-ray exposures. The resultant X-ray image is directed to the television camera 7 for its display, after conversion to an optical image by intensifier 3.

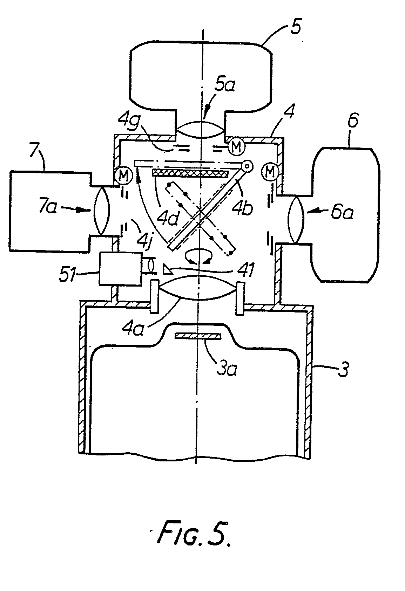

In the above described construction, the television camera 7 is so built that the television lenses 7a and 4m are disposed each in its desired position, and only its non-image-forming part is moved for performing television fluoroscopy. But as shown in Figure 5, the television camera 7 with a television lens 7a may be fixed with its optical axis coincident with the optical axis of the cinecamera 6 and a second, small-sized, television camera 5l using a charge coupled device (CCD) etc. may be fixed with its optical axis coincident with the output optical path of prism

4l; the output of television camera 5l is then displayed as an image on a television monitor only in the stereoscopic radiography mode. Although a television camera using a CCD is low in resolution, it occupies little space because of its susceptibility to miniaturization; the Figure 5 embodiment is thus simpler and smaller in construction than that of Figure 2 involving the shifting of the television camera. Particularly, when operating in the cinematography mode, when television fluoroscopy is performed only for checking the photographed images in the cinecameras 5 and 6, high resolution in the fluoroscopy mode is not required. Also, the prism 4l is sufficiently small and is so disposed in a corner of the optical path that it does not produce a shadow on the cine-film.

As described above, in the stereoscopic cinematography mode, X-rays for right and left images are alternately emitted from the X-ray tube in response to the shutter operations of the cinecameras 5 and 6 so that the right image can be photographed in one of the cinecameras and the left image in another, enabling stereo-viewing to be performed. Since a single half-mirror 4b distributes the light equally to the two cinecameras, the optical path length can be shorter than in the case when the light is distributed to one cinecamera and the television camera by employing multiple reflections. Since it is possible to minimise light loss in the half-mirror 4b and the peripheral light loss necessarily present in an optical system including a lens, there is no need to use an especially large bright lens as the primary lens 4a.

Because the half-mirror 4e and the fully reflective mirror 4d, when in operative positions for modes other than stereoscopy, occupy only the space otherwise occupied by the half-mirror 4b, the optical path lengths from the lens 4a to the cinecameras 5 and 6 can be kept to a minimum and light loss minimised. Those path lengths can also be made substantially equal thus enabling precise stereoscopic images to be obtained. In addition, it is possible to perform either single cinematography or fluoroscopy using only one mode setting and to perform television fluoroscopy while in the cinematography mode.

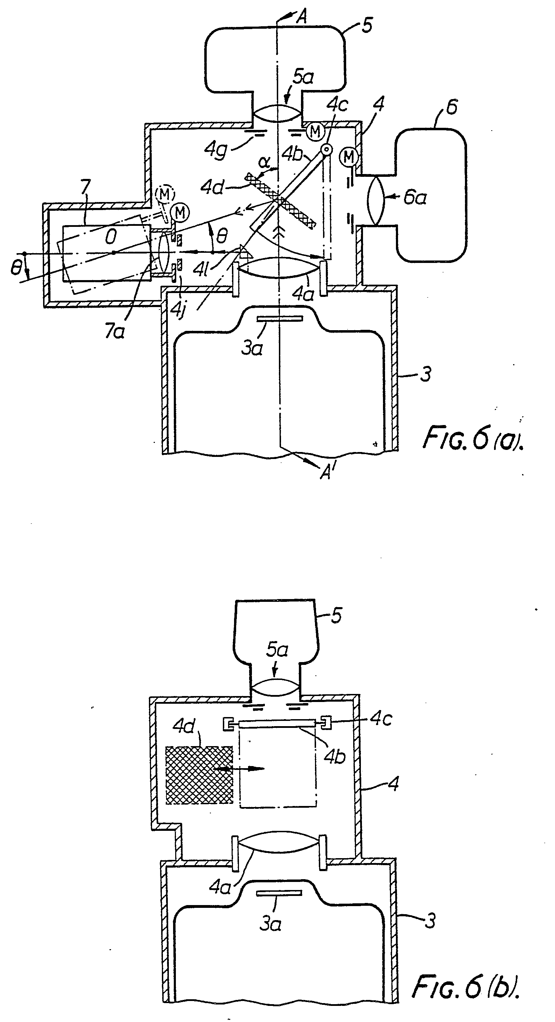

In the modified optical system of Figures 6(a) and 6(b), the 90% reflective — 10% transmissive half-mirror 4e of Figure 2 and 5 is eliminated, and only the 50% reflective — 50% transmissive half-mirror 4b and the fully reflective mirror 4d is utilized.

Although the half-mirror 4d is pivotally mounted as before, its inoperative position is now adjacent lens 6a, rather than adjacent lens 5a. When in its operative position, mirror 4b is as before arranged with its plane at an angle of 45° to the optical axis of the primary lens 4a in order to distribute the incident light image equally to the two cinecameras 5 and 6.

In the single cinematography mode, half-mirror 4b is caused to rotate about its hinge 4c to move from the optical path of the primary lens 4a and take up its inoperative position parallel with the lens 6a of the cinecamera 6. As a result, the output image from the intensifier 3 is directly applied to cinecamera 5 but is not diverted to cinecamera 6. Only cinecamera 5 is used in the single cinematography mode and receives light at a level approximately double that in the stereoscopic cinematography mode. Therefore, optical diaphragm 4g of cinecamera 5 may be remotely controlled by controller 9a so as to optimise the incident light level in each respective mode.

To enable supervisory television fluoroscopy to be performed in the cinematography mode, the small prism 4l is positioned as before within but towards one edge of the optical path of the primary lens 4a and television camera 7 is disposed with its optical axis aligned with prism 4l as shown in full line in Figure 6(a). In this embodiment, television camera 7 can be swung around the point O under the control of the controller 9a during mode setting. In the cinematography mode, the axis of television camera 7 is coincident with the output axis of prism 4l, enabling television fluoroscopy to be additionally performed. In the fluoroscopy mode, the fully reflective mirror 4d, which is otherwise positioned out of the optical path of the primary lens 4a, is brought into the optical path forming an angle α with it as shown in Figure 6(a) and reflects the output image of the image intensifier 3 into the television camera 7 which has been angularly moved by an angle θ into the position shown in chain line in Figure 6(a) in which its axis is aligned with the reflected path from mirror 4d. In this case, because the reflected light level from the fully reflective mirror 4d is greater than the light level otherwise received from prism 4l, electromotive optical diaphragm 4j positioned in front of the television camera 7 is adjusted for optimum light level. The abovementioned operation is performed by the controller 9a at the mode setting time so that the television fluoroscopy can be performed with precise resolution and at proper light level.

The action of the optical diaphragm of each cinecamera or television camera may be performed instead by an N.D. filter, a liquid crystal filter, a shutter or the like, provided it has the required degree of light level adjusting function.

## Claims

1. X-ray equipment capable of performing stereoscopic cinematography, single cinematography and fluoroscopy, the equipment comprising an X-ray tube (1) having at least a pair of focal points (fl, fr) from which X-rays are independently radiated to create an X-ray image of an object (2); means (3) for converting the X-ray image to a corresponding optical image; an optical system (4) arranged to receive and transmit the optical image, that system including a half-mirror (4b) which is substantially equally reflective and transmissive and which is movable between an operative position in which it intercepts the light path of the optical image and an inoperative position, a fully reflective mirror (4d) which is movable between an operative position in which it intercepts the light path and an inoperative position; and a small light deflector (4/) to receive light from the converting means (3); a first cinecamera (5) arranged to receive the image transmitted from the converting means (3) through the half-mirror (4b); a second cinecamera (6) arranged to receive the image transmitted from the converting means (3) and reflected by the half-mirror (4b) in its operative position, the two cinecameras having shutters which are out of phase with one another; a television camera means (7) arranged to receive the image from the converting means (3) either via the fully reflective mirror (4d) in its operative position or via the small deflector (4/); a mode setting device (8) for selecting one of the stereoscopic cinematography, single cinematography and fluoroscopy modes; and a radiographic control device (9, 9a) for controlling the positioning of the half-mirror (4b) and the fully reflective mirror (4d) according to the selected mode so that the half-mirror (4b) is brought into its operative position for stereographic cinematography to direct the image to both cinecameras (5, 6), the half-mirror (4b) is brought into its inoperative position for single cinematography, and the fully reflective mirror (4d) is brought into operative position for fluoroscopy to direct the image to the television camera means (7), the radiographic control device (9, 9a) further controlling the photographing operation of the cinecameras (5, 6) according to the selected mode, and controlling the X-ray tube (1) so that, for stereoscopic cinematography, X-rays are radiated from both focal points alternately in synchronism with the alternately open shutters of the two cinecameras (5, 6), and, for single cinematography, X-rays are radiated intermittently from one focal point in synchronism with the open state of the shutter of one cinecamera.

2. X-ray equipment according to claim 1, in which the optical system further includes a second half-mirror (4e) which is movable between a first operative position, a second operative position, and an inoperative position, and which is controlled by the radiographic control device (9, 9a) (a) to bring it to the inoperative position during stereoscopic cinematography, (b) to bring it to the first operative position for single cinematography when the image is directed to one of the cinecameras (6) by reflection at the second half mirror and to the television camera means (7) by transmission through the second half-mirror (4e), reflection at the fully reflective mirror (4d) and reflection at the second half-mirror (4e), and (c) to bring it to its second operative position for fluoroscopy when the image is directed to the television camera means (7) by reflection at the second half-mirror (4e).

3. X-ray equipment according to claim 2, in which the optical system includes a lens (4a) for directing the image from the converting means (3) along an optical axis, in which a first of the cinecameras (5) has its axis coincident with the optical axis, the other cinecamera (6) has its axis at right angles to the optical axis, and the television camera means (7) has its axis transverse to the optical axis; and the three mirrors (4b, 4d and 4e) in their operative positions intercept the optical axis.

4. X-ray equipment according to claim 3, in which the first half-mirror (4b) when in its inoperative position is between the operative position of the fully reflective mirror (4d) and the first cinecamera (5).

5. X-ray equipment according to any one of claims 2 to 4, in which the small deflector means (4/) is arranged to direct the image to the television camera means (7, 5/) when in stereographic cinematography.

6. X-ray equipment according to claim 5, in which the television camera means comprises a television camera (7) which is positionable in alignment with the second half-mirror (4e) in the single cinematography mode and in the fluoroscopy mode and in alignment with the deflector means (4/) in the stereoscopic cinematography mode.

7. X-ray equipment according to claim 5, in which the television camera means comprises a first television camera (7) fixed in position in alignment with the second half-mirror (4e), and a second television camera (5/) fixed in position in alignment with the deflector means (4/).

8. X-ray equipment according to claim 1, in which a first (5) of the cinecameras is aligned with the optical axis, the second (6) has its axis transverse to the optical axis and the television camera means (7) has its axis transverse to the optical axis, and in the single cinematography mode, the half-mirror (4b) in its inoperative position lies to one side of the optical axis whereby the image is directed without reflection to the first cinecamera (5).

9. X-ray equipment according to claim 8, in which the television camera means is a television camera (7) which is angularly adjustable to align its axis with the fully reflective mirror (4d) in its operative position during fluoroscopy and with the deflector means (4/) during cinematography.

**Patentansprüche**

1. Röntgengerät zur Durchführung stereoskopischer Kinematografie, von Einzel-Kinematografie und Fluoroskopie, gekennzeichnet durch eine Röntgenstrahlröhre (1), die wenigstens zwei Brennpunkte ($f_l$, $f_r$) aufweist, von denen Röntgenstrahlen unabhängig voneinander ausgesandt werden zur Erzeugung eines Röntgenstrahlbildes eines Objektes (2); eine Einrichtung (3) zur Umwandlung des Röntgenstrahlbildes in ein entsprechendes optisches Bild; ein optisches System zum Empfang und zur Übertragung des optischen Bildes, wobei das System einen halbdurchlässigen Spiegel (4b) aufweist, der gleiche Anteile reflektiert und durchläßt und der zwischen einer Betriebsposition im Lichtweg des optischen Bildes und einer Außerbetriebsstellung bewegbar ist; einen total reflektierenden Spiegel (4d), der zwischen einer Betriebsposition im Lichtweg und einer Außerbetriebsposition bewegbar ist; einen kleinen Lichtablenker (41), auf den Licht vom Umwandler (3) fällt; eine erste Kinokamera (5), die das vom Umwandler (3) übertragene Bild über den halbdurchlässigen Spiegel (4b) aufnimmt; eine zweite Kinokamera (6), die das vom Umwandler (6) übertragene und vom halbdurchlässigen Spiegel (4b) in seiner Betriebsstellung reflektierte Bild aufnimmt, wobei die beiden Kinokameras Verschlüsse aufweisen, die bezüglich einander außer Phase sind; eine Fernsehkamera (7), die das Bild vom Umwandler (3) entweder über den total reflektierenden Spiegel (4d) in dessen Betriebsstellung oder über den kleinen Lichtablenker (41) aufnimmt; eine Betriebsarteinstell-Einrichtung (8) zur Auswahl der Betriebsart stereoskopische Kinematographie, Einzel-Kinematographie oder Fluoroscopie, mit einer Röntgen-Steuervorrichtung (9, 9a) zur Steuerung der Positionierung des halbdurchlässigen Spiegels (4b) und des total reflektierenden Spiegels (4d) in Abhängigkeit von der ausgewählten Betriebsart, derart, daß der halbdurchlässige Spiegel (4b) in seine Betriebsstellung für stereografische Kinematografie bewegbar ist, um das Bild beiden Kinokameras (5, 6) zuzuführen, der halbdurchlässige Spiegel (4b) in seine Außerbetriebsstellung für Einzel-Kinematografie bewegbar ist und der total reflektierende Spiegel (4d) in seine Betriebsstellung für Fluoroskopie bewegbar ist, um das Bild auf die Fernsehkamera (7) zu führen, wobei die Röntgen-Steuervorrichtung (9, 9a) ferner den Aufnahmebetrieb der Kinokameras (5, 6) in Abhängigkeit von der ausgewählten Betriebsart steuert sowie auch die Röntgenstrahlröhre (1), derart, daß für stereoskopische Kinematografie Röntgenstrahlen von beiden Brennpunkten abwechselnd im Gleichgang mit den abwechselnd offenen Verschlüssen der beiden Kinokameras (5, 6) ausgestrahlt werden, und Röntgenstrahlen für Einzel-Kinematografie intermittierend von einem Brennpunkt im Gleichklang mit dem Offenzustand des Verschlusses einer Kinokamera abgestrahlt werden.

2. Röntgengerät nach Anspruch 1, dadurch gekennzeichnet, daß das optische System ferner einen zweiten halbdurchlässigen Spiegel (4e) aufweist, der zwischen einer ersten Betriebsstellung, einer zweiten Betriebsstellung und einer Außerbetriebsstellung bewegbar ist und der durch die Röntgen-Steuervorrichtung (9, 9a) steuerbar ist, um den Spiegel (a) während der stereoskopischen Kinematografie in die Außerbetriebsstellung zu bewegen, (b) für Einzel-Kinematografie in die erste Betriebsstellung zu bewegen, wenn das Bild einer der Kinokameras (6) durch Reflexion am zweiten halbdurchlässigen Spiegel und der Fernsehkamera (7) zugeführt wird durch Übertragung durch den zweiten halbdurchlässigen Spiegel (4e), durch Reflexion am total reflektierenden Spiegel und Reflexion am zweiten halbdurchlässigen Spiegel (4e), und (c) in seine zweite Betriebsstellung für Fluoroskopie zu bewegen, wenn das Bild der Fernsehkamera (7) durch Reflexion am zweiten halbdurchlässigen Spiegel (4e) zugeführt wird.

3. Röntgengerät nach Anspruch 2, dadurch gekennzeichnet, daß das optische System eine Linse (4a) aufweist, um das Bild von dem Umwandler (3) entlang einer optischen Achse zu führen, in der die Achse einer der Kinokameras (5) mit der optischen Achse zusammenfällt, die Achse der anderen Kinokamera (6) rechtwinklig zur optischen Achse verläuft und die Achse der Fernsehkamera (7) quer zur optischen Achse verläuft, und daß die drei Spiegel (4b, 4d und 4e) in ihren Betriebsstellungen die optische Achse schneiden.

4. Röngengerät nach Anspruch 3, dadurch gekennzeichnet, daß der erste halbdurchlässige Spiegel (4b) sich in seiner Außerbetriebsstellung zwischen der Betriebsstellung des total reflektierenden Spiegels (4d) und der ersten Kinokamera (5) befindet.

5. Röntgengerät nach einem der Ansprüche 2 bis 4, dadurch gekennzeichnet, daß der kleine Lichtumlenker (41) bei der stereoskopischen Kinematografie das Bild der Fernsehkameraeinrichtung (7, 51) zuführt.

6. Röntgengerät nach Anspruch 5, dadurch gekennzeichnet, daß die Fernsehkameraeinrichtung eine Fernsehkamera (7) umfaßt, die mit dem zweiten halbdurchlässigen Spiegel (4e) ausrichtbar ist in der Einzel-Kinematografie-Betriebsart und in der Fluoroskopie-Betriebsart und mit dem Lichtumlenker (41) in der stereoskopischen Kinematografie-Betriebsweise ausrichtbar ist.

7. Röntgengerät nach Anspruch 5, dadurch gekennzeichnet, daß die Fernsehkameraeinrichtung eine erste ortsfeste Fernsehkamera (7) in Ausrichtung mit dem zweiten halbdurchlässigen Spiegel (4e) aufweist und eine zweite ortsfeste Fernsehkamera (51) in Ausrichtung mit dem Lichtablenker (41).

8. Röntgengerät nach Anspruch 1, dadurch gekennzeichnet, daß eine (5) der Kinokameras mit der optischen Achse ausgerichtet ist, daß die Achse der zweiten Kinokamera (6) und der Fernsehkamera (7) quer zur optischen Achse angeordnet sind, und daß in der Einzel-Kinematografie-Betriebsart der halbdurchlässige Spiegel (4b) in seiner Außerbetriebsstellung neben der optischen Achse liegt, wodurch das Bild ohne Reflexion der ersten Kinokamera (5) zuführbar ist.

9. Röntgengerät nach Anspruch 8, dadurch gekennzeichnet, daß die Fernsehkameraeinrichtung eine erste Fernsehkamera (7) ist, die winkelverstellbar ist zur Ausrichtung ihrer Achse mit dem total reflektierenden Spiegel (4d) in dessen Betriebsstellung für Fluoroskopie und mit dem Lichtumlenker (41) für Kinematografie.

**Revendications**

1. Equipement radiologique capable d'effectuer des opérations de cinématographie stéréoscopique, de cinématographie monoscopique et de fluoroscopie, équipement qui comprend un tube à rayons (1) X ayant, au moins, deux points focaux (fl, fr) desquels des rayons X sont émis séparément afin de créer une image radiologique d'un objet (2); des moyens (3) pour couvertir cette image radiologique en une image optique; un système optique conçu et réalisé pour recevoir et transmettre cette image optique, ce système incluant un miroir semi-réfléchissant (4b) qui est sensiblement réfléchissant et transparent de manière égale et qui peut se déplacer entre une position active dans laquelle il coupe la trajectoire de la lumière de l'image optique, et une position inactive, un miroir totalement réfléchissant (4d) qui peut se déplacer entre une position active dans laquelle il coupe la trajectoire de la lumière et une position inactive et un petit déflecteur de lumière (41) pour recevoir la lumière des moyens de conversion (3); une première caméra cinématographique (5) conçue et réalisée pour recevoir l'image transmise par les moyens de conversion (3) à travers le miroir semi-réfléchissant (4b); une seconde caméra cinématographique (6) conçue et montée pour recevoir l'image transmise par les moyens de conversion (3) et qui a été réfléchie par le miroir semi-réfléchissant (4b) dans sa position active, les deux caméras cinématographiques ayant des obturateurs qui sont mutuellement déplacés; une caméra de télévision (7) disposée pour recevoir l'image des moyens de conversion (3) soit du miroir totalement réfléchissant (4d) dans sa position active, soit du petit déflecteur (41); un dispositif de réglage de mode de fonctionnement (8) pour sélectionner soit la cinématographie stéréoscopique, soit la cinématographie monoscopique ou la fluoroscopie; et un dispositif de commande radiographique (9, 9a) pour régler la position du miroir semi-réfléchissant (4b) et du miroir totalement réfléchissant (4d) en accord avec le mode choisi de sorte que le miroir semi-réfléchissant (4b) est transféré dans sa position active pour opérer en cinématographie stéréoscopique afin de diriger l'image vers les deux caméras (5, 6), le miroir semi-réfléchissant étant transféré dans sa position inactive pour opérer en cinématographie monoscopique, tandis que le moroir totalement réfléchissant (4d) est amené en position active pour opérer en fluoroscopie afin de diriger l'image vers la caméra de télévision (7), le dispositif de commande radiographique (9, 9a) commandant, en outre, le fonctionnement des caméras cinématographiques (5, 6) en accord avec le mode choisi, et commandant le tube à rayons X (1) de manière que, pour la cinématographie stéréoscopique, les rayons X sont émis alternativement par les deux points focaux en synchronisme avec l'ouverture alternative des obturateurs des deux caméras (5, 6), tandis que pour la cinématographie monoscopique, les rayons X sont émis, de façon intermittente par l'un des points focaux, en synchronisme avec l'action d'ouverture de l'obturateur de l'une des caméras cinématographiques.

2. Equipement radiologique selon la revendication 1, caractérisé en ce que le système optique comprend, en outre, un second miroir semi-réfléchissant (4e) qui peut se déplacer entre une première position active, une seconde position active et une position inactive, et qui est commandé par le dispositif de commande radiographique (9, 9a) à se placer (a) dans sa position inactive pendant la cinématographie stéréoscopique, à se placer (b) dans sa première position active pour opérer en cinématographie monoscopique quand l'image est dirigée vers l'une des caméras (6) par réflexion sur le second miroir semi-réfléchissant et vers la caméra de télévision (7) à travers le second miroir semi-réfléchissant (4e) et par réflexion sur le miroir totalement réfléchissant (4d) et sur le second miroir semi-réfléchissant (4e) et (c) à se placer dans sa seconde position active pour la fluoroscopie quand l'image est dirigée vers la caméra de télévision (7) par réflexion sur le second miroir semi-réfléchissant (4e).

3. Equipement radiologique selon la revendication 2, caractérisé en ce que le système optique comporte un objectif (4a) pour diriger l'image issue des moyens de conversion le long d'un axe optique, en ce que l'axe optique de la première caméra cinématographique (5) coïncide avec l'axe optique, en ce que l'axe de l'autre caméra cinématographique (6) est perpendiculaire à l'axe optique, et en ce que l'axe de la caméra de télévision est transversal à l'axe optique; et en ce que, en position active, les trois miroirs (4b, 4d et 4e) coupent cet axe optique.

4. Equipement radiologique selon la revendication 3, caractérisé en ce que, dans sa position inactive, le premier miroir semi-réfléchissant (4b) est placé entre la position active du premier miroir totalement réfléchissant (4d) et la première caméra cinématographique (5).

5. Equipement radiologique selon l'une quelconque des revendications précédentes 2 à 4, caractérisé en ce que le petit déflecteur (41) est disposé de manière à diriger l'image vers la caméra de télévision (7, 51) dans le mode cinématographique stéréoscopique.

6. Equipement radiologique selon la revendication 5, caractérisé en ce que la caméra de télévision (7) peut être placée dans l'alignement du second miroir semi-réfléchissant (4e) quand on opère en cinématographie monoscopique et peut être alignée avec le déflecteur (41) en cinématographie stéréoscopique.

7. Equipement radiologique selon la revendication 5, caractérisé en ce qu'il comprend une première caméra de télévision (7) fixée dans l'alignement du second miroir semi-réfléchissant (4e), et une seconde caméra de télévision (51) fixée dans l'alignement du déflecteur (41).

8. Equipement radiologique selon la revendication 1, caractérisé en ce que la première caméra cinématographique (5) est alignée avec l'axe optique, en ce que l'axe de la seconde caméra (6) est transversal à l'axe optique, tandis que l'axe de la caméra de télévision (7) est aussi transversal à l'axe optique, et en ce que, dans le mode de fonctionnement en cinématographie, monoscopique, le miroir semi-réfléchissant (4b) est situé, dans sa position inactive, sur un côté de l'axe optique, ce que fait que l'image est dirigée sans réflexion vers la première caméra cinématographique.

9. Equipement radiologique selon la revendication 8, caractérisé en ce qu'il comprend une caméra de télévision (7) qui est réglable angulairement afin de pouvoir aligner son axe avec le miroir totalement réfléchissant (4d) dans sa position active en fluoroscopie et avec le déflecteur (41) en cinématographie.

0 052 995

*Fig. I.*

Fig. 2 (a).

Fig. 2 (b).

FIG. 3.

*(a)* SHUTTER SIGNALS FROM CINECAMERA 5

*(b)* X-RAY PULSES FOR THE RIGHT

*(c)* SHUTTER SIGNALS FROM CINECAMERA 6

*(d)* X-RAY PULSES FOR THE LEFT

*(e)* FILM OF CINECAMERA 5

*(f)* FILM OF CINECAMERA 6

FIG. 4.

FIG.5.

Fig. 6 (a).

Fig. 6 (b).